# EUROPEAN PATENT APPLICATION

(11) **EP 2 070 557 A1**
(43) Date of publication of application: **17.06.2009**
(21) Application number: 07024146.8
(22) Date of filing: 12.12.2007
(51) Int. Cl.: A61L 27/20, A61L 24/08, A61F 2/28

(54) **Implantable microbial cellulose materials for hard tissue repair and regeneration**

(71) Applicant: Xylos Corporation, Langhorne, PA 19047 (US)
(72) Inventor: Serafica, Gonzalo, Langhorne, PA 19047 (US); Ace, Constance, Whitehouse, NJ 08888 (US); Oberholtzer, Lauren, Philadelphia, PA 19130 (US)
(74) Representative: HOFFMANN EITLE

(57) **Abstract**

An implantable microbial cellulose-containing material is provided which is in a moldable paste form and has suitable implantation properties for repair or replacement of hard tissue. Also provided is a use of the moldable paste containing microbial cellulose for the manufacture of an agent for promoting hard tissue growth.

## Description

### BACKGROUND OF THE INVENTION

### Field of Invention

This invention relates to implantable microbial cellulose-containing materials, and more particularly to moldable pastes containing microbial cellulose having suitable implantation properties for repair or replacement of hard tissue. The invention also relates to the use of the moldable pastes containing microbial cellulose for the manufacture of an agent for promoting hard tissue growth.

### Description of the related art

Various materials used as implantable devices in the medical industry have been well documented and can be divided into biologic, synthetic and biosynthesized. Biologic materials used as bone void fillers include autograft tissue (a patient's own tissue), allograft (tissue from another individual of the same species) and xenograft (tissue from another species). To fill bony defects, autograft remains the gold standard, however, the harvest of tissue from one part of a body to be implanted into another part carries a degree of morbidity; often, there is more postoperative pain at the harvest site than at the implant site. Allograft, as described in U.S. Patent Nos. 5,073,373; 5,290,558; 5,510,396; 6,030,635; and 6,755,863, has been used as a medical implant for a variety of indications including as a bone graft substitute. Often there is limited availability of the material and there is a risk of disease transmission from graft to host. Xenograft, which includes animal collagen, has been implanted as bone graft substitutes (U.S. Patent No. 5,830,493) and for use in other tissue repair and replacement (U.S. Patent Nos. 5,810,855; 6,179,872; and 6,206,931). These materials carry the risk of disease transmission from donor to host and in addition, are often cross-linked using cytotoxic chemicals to improve their mechanical strength and degradation profile.

Synthetic materials, which include polymers comprised of polylactic acid (PLA), polyglycolic acid (PGA), and polypropylene, have long been used as surgical sutures. These synthetic materials have been fabricated into films, mesh, and more complex three dimensional structures depending on the intended applications as described in U.S. Patent Nos. 5,441,508; 5,830,493; 6,031,148; 6,852,330; and 6,946,003.

Additional synthetic matrices consist of calcium based materials such as calcium phosphate in the form of hydroxyapatite, dicalcium phosphate or tricalcium phosphate; calcium carbonate in the form of calcite or aragonite; calcium sulfate, calcium aluminate and composites of these materials. These matrices demonstrate variable resorption rates and characteristics. Other bone graft substitute materials include bioactive glasses and glass ceramics.

These synthetic materials possess certain physical characteristics that make them suitable as an implant material. Such properties include biocompatibility, strength, and chemical stability which can be particularly important for a specific application.

However, these synthetic materials also have limitations and disadvantages such as a limited range of physical and biochemical properties, unfavorable degradation products and profiles, leaching of chemicals, difficult handling properties, and binding of proteins meant to be released. Thus, there remains a need to explore alternative materials more suitable for specific surgical applications.

Biosynthetic materials have also been used for tissue repair and augmentation. Chitosan, for instance, can be considered biosynthetic in that it is produced by living organisms, i.e. certain shellfish. This material has been suggested for use in various medical implantable applications that include scaffolds for bone and soft tissue regeneration.

Another biomaterial that has had extensive use for surgical applications is cellulose and the use of viscose or regenerated cellulose as implantable articles. Several investigators have studied tissue biocompatibility of cellulose and its derivatives (Miyamoto, T. et al., Tissue Biocompatibility of Cellulose and its derivatives. J. Biomed. Mat. Res., V. 23, 125-133 (1989)) and examined some specific applications for the material. The oxidized form of regenerated cellulose has long been used as a hemostatic agent and adhesion barrier (Dimitrijevich, S. D., et al. In vivo Degradation of Oxidized regenerated Cellulose. Carbohydrate Research, V. 198, 331-341 (1990), Dimitrijevich, S. D., et al. Biodegradation of Oxidized regenerated Cellulose Carbohydrate Research, V. 195, 247-256 (1990)) and is known to degrade much faster than the non-oxidized counterpart. A cellulose sponge studied by Martson, et al., showed excellent biocompatibility with bone and connective tissue formation during subcutaneous implantation (Martson, M., et al., Is Cellulose sponge degradable or stable as an implantation material? An in vivo subcutaneous study in rat. Biomaterials, V. 20, 1989-1995 (1999), Martson, M., et al., Connective Tissue formation in Subcutaneous Cellulose sponge Implants in rats. Eur. Surg. Res., V. 30, 419-425 (1998), Martson, M., et al., Biocompatibility of Cellulose Sponge with Bone. Eur. Surg. Res., V. 30, 426-432 (1998)). The authors surmised that cellulose material can be a viable long term stable implant. Other forms and derivatives of cellulose have also been investigated (Pajulo, O. et al. Viscose cellulose Sponge as an Implantable matrix: Changes in the structure increase production of granulation tissue. J. Biomed. Mat. Res., V. 32, 439-446 (1996), Mello, L. R., et al., Duraplasty with Biosynthetic Cellulose: An Experimental Study. Journal of Neurosurgery, V. 86, 143-150 (1997)).

However, the prior art mentions only limited applications of microbial cellulose. For example, the use of microbial cellulose in the medical industry has been described for liquid loaded pads (U.S. Patent No. 4,588,400), skin graft or vulnerary covers (U.S. Patent 5,558,861), wound dressings (U.S. Patent No. 5,846,213) and topical applications (U.S. Patent No. 4,912,049). These patents have focused on the use of microbial cellulose for topical applications and have not cited its particular application as an implantable material. Mello et al., described above, suggest the use of microbial cellulose as a soft tissue dura replacement, but not for bony areas. The only patent that describes the use of microbial cellulose obtained from *Acetobacter xylinum* as an implant is U.S. Patent No. 6,599,518 wherein a solvent dehydrated microbially derived cellulose material can be used specifically for tissue repair materials, tissue substitutes and bulking agents for plastic and reconstructive surgical procedures. The materials described by the '518 patent differ from the present invention in that they possess physical characteristics such as minimal elongation and high rigidity. These attributes render the implant material non-conformable and therefore not useful for particular surgical applications such as hard tissue repair or replacement. The '518 patent does not specify processing methods other than solvent dehydration at ambient pressure to produce implantable products. This differs from the present invention that describes either a wet paste form of microbial cellulose or drying using a method that allows the material to be moldable to fit into irregular shaped bony defects. The form of the material in '518 allows for only minimal absorption of liquid and therefore only minimal swelling. The present invention can have a varied fiber size depending on the processing and can therefore absorb liquid, swell to fill a space or have minimal swelling, and increased conformability over the '518 material.

Prior art suggests that a bone void filler with an active agent is preferred. Damien and Parsons [Damien C. J. and Parsons J.R. Bone graft and bone graft substitutes: A review of current technology and applications J Appl. Biomat Vol 2 Pages 187-208 (1991)] describe a variety of materials for use as carriers for bone forming growth factors. These include collagen, hydroxyapatite, tricalcium phosphate, bioglasses and polymers such as polylactic acid and polyglycolic acid. They conclude that collagen may be an essential ingredient in the makeup of a carrier for optimal osteoinductive expression of osteoinductive agents. In their review there is no mention of cellulose, especially microbial cellulose and their conclusions suggest that any effective bone graft substitute with an active agent would require collagen.

Similarly, U.S. Patent 5,563,124 describes an osteogenic product and process comprising calcium carbonate and a bone growth factor. Included are materials that also contain collagen, fibrin and alginate, but no mention of microbial cellulose is made.

In U.S. Patent 5,558,861, the use of microbially-produced cellulose gel as a skin graft or vulnerary cover was described. The gel is in a form of a sheet of microbial cellulose which has been chemically modified and subjected to collagen treatment to enhance its ability to culture various types of cells. It did not disclose using the material as an orthopedic matrix material that can be loaded with useful agents such as BMP's. The patent also failed to disclose the use of microbial cellulose as an implantable medical device.

An amorphous non-sheeted gel of microbial cellulose is described by Serafica et al. in U.S. Patent Application 20040142019. In this application the amorphous gel is described as a wound dressing to which various agents could be added. Knowing that agents, including bone morphogenetic proteins, have demonstrated efficacy in the wound healing, this factor was included. The application does not disclose any use of the gel or gel with factors in the repair of hard tissue when implanted into the body.

Microbial cellulose for use in dura replacement is described by Damien et al. in U.S. Patent Application 20050042263. This material differs from the current invention in its application and in its composition: the dura material is in sheet form and can either be used as an onlay or sutured into place to repair soft tissue, while the material in the current invention is in a putty, gel or paste form that can be molded or packed into hard tissue defects for the repair of bone. The microbial cellulose in the above dura application has intact fibers, while those in the current invention are either chopped or grown in a non-fibrous form. The material in the dura application has mechanical strength characteristics so as to hold a suture and/or to achieve a water-tight closure, while the current invention will not need to hold a suture and there is no required mechanical strength. The current invention should calcify and form bone, while the material in the dura application should not demonstrate any calcification at the dural site. The current application may be a composite with calcium salts, polymers, etc. and may deliver active agents; the dura application does not describe a composite or delivery of active agents.

Microbial cellulose for use in bone repair as a porous matrix combined with calcium salts is described by Hutchens et al. [U.S. Patent Application 20040096509]. As described, their material is a porous polymer matrix that requires a minimum Young's modulus of 10GPa and a calcium salt within some of the pores.

Accordingly, heretofore, there has not been provided an acceptable implantable material comprising microbial cellulose for use in hard tissue repair, regeneration or replacement applications.

### SUMMARY OF THE INVENTION

It is an object of the present invention to provide a new class of implantable materials utilizing microbial cellulose that has desirable physical and biochemical properties for use in hard tissue repair, regeneration and replacement including bone void or defect filling and spine fusion.

To achieve the above object, the present invention provides a moldable paste containing microbial cellulose with a microbial cellulose content of 1 to 99 % by weight, and a method of obtaining said paste. In a preferred embodiment, the microbial cellulose may be modified by oxidation to make it bioresorbable. In another preferred embodiment, the moldable paste has a stiffness less than 150 N.

The material in the present invention is in a different form than that envisioned by Hutchins: it is a paste, gel or putty form that is moldable to fit into various sizes and shapes of bony defects. As such, the present invention has no strength requirement and will not experience tensile loading; therefore, there is no Young's modulus requirement. Also in the present invention, there is no necessity of having a calcium salt with the microbial cellulose. Rather it can be implanted as processed or by adding calcium salts into the paste.

A preferred embodiment of the present invention provides a moldable paste of microbial cellulose containing an ingredient for flow modification to modify the microbial cellulose composition with desirable flow properties. The ingredient for flow modification may be present in an amount of 0.1 to 30 % by weight in the paste. Preferable ingredients for flow modification include glycerol, polyethylene glycol, Tween, chitosan, acrylates, ethyl cellulose, hydroxypropyl cellulose and carboxymethyl cellulose.

Another preferred embodiment of the present invention provides a moldable paste with suitable stiffness and conformability properties for implantation. More specifically, the moldable paste of the present invention contains a calcium salt in an amount of 5 to 90 % by weight in the paste with or without the ingredient for flow modification. Preferably, the calcium salt is one or more of hydroxyapatite, tricalcium phosphate (α and β phases) and calcium sulfate.

In another preferred embodiment, the present invention provides a moldable paste of microbial cellulose containing at least one medically useful agent for promoting hard tissue growth, where the agent is released in a controlled manner by the microbial cellulose. The agent is present preferably in an amount of 0.1 to 10% by weight in the paste as processed or in the paste containing the above described ingredient for flow modification, calcium salt or combination thereof. Any number of medically useful agents for tissue repair may be used, which include antimicrobial agents, drugs and growth factors.

Another aspect of the present invention provides the use of these moldable pastes for the manufacture of an agent for promoting hard tissue growth. Preferably, the agent is for bone void filling or a bone graft substitute.

### DESCRIPTION OF FIGURES

Figure 1 is a graph showing the release of Bovine Serum Albumin from a microbial cellulose pad over a 72-hour period.
Figure 2 shows stiffness values for microbial cellulose paste samples containing 5% and 10% microbial cellulose with an addition of hydroxyapatite (HA), where the samples containing 5% microbial cellulose were prepared with processing times of one and five minutes.
Figure 3 shows BSA release profiles from microbial cellulose paste samples containing 5% and 10% microbial cellulose, where the samples containing 5% microbial cellulose were prepared with processing times of one and five minutes.
Figure 4 shows PHMB release profiles from microbial cellulose paste samples containing 5% and 10% microbial cellulose, where the samples containing 5% microbial cellulose were prepared with processing times of one and five minutes.
Figure 5 shows fluid absorption profiles of microbial cellulose paste samples containing 5% and 10% microbial cellulose, where the samples containing 5% microbial cellulose were prepared with processing times of one and five minutes.
Figure 6 shows fluid donation profiles of microbial cellulose paste samples containing 5% and 10% microbial cellulose, where the samples containing 5% microbial cellulose were prepared with processing times of one and five minutes.
Figure 7 shows effects of drying techniques on the stiffness of microbial cellulose paste samples.

### DETAILED DESCRIPTION OF THE PREFERRED EMBODIMENTS

The present invention describes an implantable material comprised of microbial cellulose. The instant implantable material has properties necessary for *in vivo* applications. For example, the implantable material of the present invention is in a paste, gel or putty form that is moldable into a desired three-dimensional form and possesses desired stiffness and conformability characteristics for a variety of medical and surgical applications including hard tissue repair.

The implantable materials of the present invention are comprised of microbial cellulose. Those methods of preparing microbial cellulose are known to those of ordinary skill and are described, for example, in U.S. Patent Nos. 5,846,213 and 4,912,049. Any cellulose producing organism can be used in producing the raw biosynthetic cellulose material. However, biosynthetic cellulose pellicles produced from a static culture of *Acetobacter xylinum* are preferred.

The microbial cellulose content of the pellicle is dependent on the amount of media supplied to the A.x. bacteria. Once the pellicle is harvested, the excess medium contained in it is removed by mechanical compression, then the pellicle is subjected to chemical cleaning to convert the raw microbial cellulose film into a medical grade and non-pyrogenic implantable material. The cleaned pellicle is mechanically pressed to reduce the water content, resulting in a microbial cellulose pad which later may be cut to shape and physically processed by chopping, grinding or milling to a paste consistency that is moldable. The moldable paste may be further subjected to a solvent exchange with methanol, then may be dried. Typical microbial cellulose contents in the moldable paste of the present invention ranges from 1 to 99 % by weight, preferably 2 to 65 % by weight, more preferably 3 to 20 % by weight.

Physical properties of the moldable paste such as conformability and stiffness are important factors to assess implantable characteristics of the moldable paste of the present invention. These properties may be assessed by commonly utilized techniques such as scanning electron microscopy (SEM), mechanical testing or other standard physical tests. For example, stiffness of the moldable paste may be evaluated using ASTM test method D 4032 for testing stiffness of fabrics. The stiffness value of the moldable paste of the present invention is preferably less than 150N and more preferably less than 80N, to be suitable for implantation. Chemical properties such as degree of crystallinity, active chemical groups and degree of polymerization are also considerable factors and may be examined by techniques such as x-ray crystallography and infrared spectroscopy.

In a preferred embodiment, the invention provides a moldable paste containing microbial cellulose that is bioresorbable. Variable degrees of oxidation affect the rate of resorption. For example, the microbial cellulose may be subjected to oxidation at various degrees to render it bioresorbable. This resorption can be tailored to the rate of bone formation so that voids are not created by the material degrading too quickly. Preferably, the degree of oxidation is between 10 and 30 %.

The degree of oxidation of the microbial cellulose is achieved by varying a factor such as concentration and volume of oxidizing agent, a ratio of oxidizing agent to microbial cellulose, reaction temperature and duration, and combination thereof. For example, the molarity of oxidizing agent may be in the range of 0.005M to 0.5M, and the temperature may be from about 5 to 50°C. Preferably, the microbial cellulose may be oxidized for at least 30 minutes, or 1, 6, 12, or 24 hours, or longer. In a preferred embodiment, the microbial cellulose may be oxidized with a solution containing sodium periodate and optionally, a salt such as NaCl. In a specific embodiment, the microbial cellulose paste may be oxidized by incubating the paste in 80 mM NAlO₄ for 18 to 20 hrs at 30°C.

The microbial cellulose contained in the moldable paste may be modified by the addition of an ingredient for flow modification in order to control water absorption and pliability which are desirable properties for implantable materials. Such ingredient includes, but not limited to, glycerol, polyethylene glycol, Tween, chitosan, acrylates, ethyl cellulose, hydroxypropyl cellulose or carboxymethyl cellulose. In an especially preferred embodiment, the ingredient for flow modification is hydroxypropyl cellulose.

The amount of the ingredient for flow modification ranges from 0.1 to 40% by weight, preferably 1 to 30 % by weight, more preferably 5 to 25 % by weight, in the moldable paste. In an especially preferred embodiment, the amount of the ingredient for flow modification is 1 to 5 % by weight, which provides a moldable paste with moderate formability and excellent water retention properties.

The stiffness of the moldable paste may be manipulated by the addition of a calcium salt. The calcium salt to be used includes hydroxyapatite (HA), tricalcium phosphate (TCP) and calcium sulfate. The calcium salt may be present in an amount of 5 to 95 % by weight, preferably 5 to 90 % by weight, more preferably 5 to 80 % by weight, in the moldable paste of the present invention.

In a preferred embodiment, the moldable paste is oxidized before being modified with the calcium salt.

The present invention also contemplates a moldable paste of microbial cellulose containing at least one medically useful agent. Any number of medically useful agents for tissue repair can be used in the invention. A medically useful agent is one having therapeutic, healing, curative, restorative or medicinal properties. The agent is medically useful if it reduces inflammation, increases cell attachment, recruits cells, and/or causes differentiation of the cells to repair the damaged tissue. Such medically useful agents include collagen and insoluble collagen derivatives, and soluble solids and/or liquids dissolved therein. Also included are amino acids, peptides, vitamins, co-factors for protein synthesis; hormones; endocrine tissue or tissue fragments; synthesizers; enzymes such as collagenase, peptidases, oxidases; cell scaffolds with parenchymal cells; angiogenic drugs and polymeric carriers containing such drugs; collagen lattices; biocompatible surface active agents, antigenic agents; cytoskeletal agents; cartilage fragments, living cells such as chondrocytes, bone marrow cells, mesenchymal stem cells, natural extracts, tissue transplants, bioadhesives, transforming growth factor (TGF-beta) and associated family proteins (bone morphogenetic protein (BMP), growth and differentiation factors (GDF) etc.), fibroblast growth factor (FGF), insulin-like growth factor (IGF-1) and other growth factors; growth hormones such as somatotropin; bone digestors; antitumor agents; fibronectin; cellular attractants and attachment agents; immuno-suppressants; permeation enhancers; and peptides, such as growth releasing factor, P-15 and antimicrobial agents. In a preferred embodiment, the medically useful agent is a BMP (BMP-2 or BMP-7).

The agent may be in its free base or acid form, or in the form of salts, esters, or any other pharmacologically acceptable derivatives, enantomerically pure forms, tautomers or as components of molecular complexes. These agents may be added to a composition containing the microbial cellulose carrier, either at any step in the manufacturing process or directly to the final moldable paste. The amount of agent to be incorporated in the moldable paste varies depending on the particular agent, the desired therapeutic effect, and the time span for which the moldable paste is to provide therapy. For purposes of the invention, the amount of agent in the system can vary from about 0.0001 % by weight to as much as 60 % by weight, preferably from 0.1 to 10 % by weight, more preferably 1 to 5 % by weight.

The present invention contemplates a moldable paste with a variety of combination in which microbial cellulose may be combined with more than one of the above described substances, i.e. the ingredient for flow modification, calcium salt and medically useful agent, in their corresponding preferred amounts in the moldable paste. In a preferred embodiment, the moldable paste is prepared from a microbial cellulose paste containing microbial cellulose 5% by weight. Preferably, the moldable paste contains microbial cellulose in an amount of 10 to 40 % by weight, PEG, carboxymethyl cellulose or hydroxylpropyl cellulose in an amount of 0.1 to 30 % by weight, more preferably 5 to 25 % by weight, and TCP or HA in an amount of 5 to 95 % by weight, more preferably 40 to 80 % by weight. The microbial paste may be oxidized before being modified with the above described substances. In another preferred embodiment, a medically useful agent such as Bone Morphogenetic protein (BMP-2 or BMP-7) may be further added to the moldable paste of microbial cellulose containing ingredient for flow modification, calcium salt or combination thereof. The amount of such agent is preferably 0.01 to 10 % by weight, more preferably 0.1 to 5 % by weight, in the paste.

In still another preferred embodiment, the present invention provides a method for producing a moldable paste containing microbial cellulose for a variety of medical and surgical applications. The method comprises the steps of preparing a microbial cellulose pellicle, forming a paste by chopping, grinding or milling the pellicle, subjecting the paste to a solvent exchange process and subsequently a drying process. For example, a pellicle prepared from a culture of A.x. bacteria may be mechanically pressed and the resulting pellicle pad may be placed in a blender with additional water to form a paste. Methanol is preferably used for the solvent exchange process. Carbon dioxide is preferably used for supercritical drying.

Yet another embodiment is the use of the moldable paste of the present invention for the manufacture of an agent for promoting hard tissue growth. Preferably, the agent is for bone void filling or a bone graft substitute. The agent can be applied in any bony site in the skeleton to fill various sized bony defects, and can be used in bony surgical applications in the orthopaedic, dental, periodontal, and craniomaxillofacial fields.

Preferably, the moldable paste of the present invention will be further sterilized for applications as medical implantable articles using standard sterilization methods such as gamma irradiation, e-beam irradiation, ethylene oxide or steam sterilization. The biocompatibility/safety properties of the moldable paste of the present invention may be assessed *in vitro* and *in vivo* testing.

In a preferred embodiment, the present invention provides a method of bone defect filling. The method comprises the steps of preparing a moldable paste containing microbial cellulose and implanting said paste into a subject in need thereof. For example, the present invention may be easily prepared as a dry or hydrated paste for direct application into bone voids. For this application the paste must be conformable so as to fill the site and remain without dislocating.

The efficacy of bone defect filling may be investigated by evaluating the moldable paste for both its osteoconductive and osteoinductive potential. In the evaluation, animals may be implanted with moldable pastes derived from both resorbable and non-resorbable microbial cellulose. Each animal may have defects created in them. With histological analysis, each implant site may be evaluated for new bone formation, residual implant material and any evidence of pathologic response for each of the moldable paste.

The present invention also contemplates a method for preparing an implantable device for medical and surgical applications. The method comprises the steps of providing a moldable paste containing microbial cellulose and incorporating said paste into an implantable device for medical and surgical applications. Once produced, the microbial cellulose may be incorporated or fashioned into medical devices by commonly known methods such as molding, cross-linking, chemical surface reaction, dehydrating and/or drying, cutting or punching. Such medical devices include tissue substitutes or scaffolds for repair or reinforcement of damaged hard tissue. For example, the instant moldable paste may be used as a scaffold in tissue engineering, substitution and replacement for hard tissue such as bone.

Yet another preferred embodiment, the present invention provides a method for use of the implantable device as an adjunct to achieve spine fusion. For this application the device must be easily implanted and remain *in situ.* Depending on the surgery the device can be implanted alone or as a filler for spine cage implants or cadaveric femoral ring implants.

The properties of the moldable microbial cellulose-based paste may be compared to a wide variety of implantable materials available including calcium salts, polymers, collagen, and human or animal derived tissue currently being used in the medical industry. Based on the results of these comparisons, including conformability, bone forming capacity and resorption rate, a number of moldable cellulose-based paste articles may be tailored for specific applications.

The present invention will be illustrated through the following examples.

### Example 1:

### Implantable Microbial cellulose Preparation

To prepare the microbial cellulose of the invention, *Acetobacter xylinum* microorganisms are cultured in a bioreactor containing a liquid nutrient medium at 30 degrees Celsius at an initial pH of 3-6. The medium is based on sucrose or other carbohydrates.

The bioreactor is composed of a plastic box fitted with an airtight cover. Dimensions of the bioreactor measured 3.5 in x 3.5 in. An aeration port is made in the bioreactor that allows the proper oxygen tension to be achieved.

The fermentation process under static conditions is allowed to progress for a period of about 10-14 days, during which the bacteria in the culture medium produce an intact cellulose pellicle. Once the media is expended, the fermentation is stopped and the pellicle removed from the bioreactor. This material is termed '250'.

### 1. Processing and Depyrogenation Procedures

The excess medium contained in the pellicle is removed by mechanical compression prior to chemical cleaning and subsequent processing of the pellicle. The microbial cellulose pellicle is subjected to a series of chemical wash steps to convert the raw microbial cellulose film into a medical grade and non-pyrogenic implantable material. Processing starts with an 8% sodium hydroxide solution at 70-75 degrees Celsius for 1 hour, followed by a rinse in deionized water and then a soak in 0.25% hydrogen peroxide at 70-75 degrees Celsius for 1 hour.

The resulting films can be tested for pyrogens and mechanical properties. The amount of cellular debris left in the microbial cellulose pad after processing is measured by validated Limulus Amoebocyte Lysate (LAL) testing as outlined by the U.S. Food and Drug Administration (FDA) in 21 CFR10.90. The instant cleaning process outlined above provides a nonpyrogenic microbial cellulose pad (≤0.50 EU/ml). The steps of the LAL test are defined by the test kit manufacturer and can simply be followed to yield the pyrogen level in the microbial cellulose film.

### 2. Final product processing

Once cleaned, the pellicles can be mechanically pressed to reduce the water content. The resulting pad is cut to shape and packaged.

### Example 2:

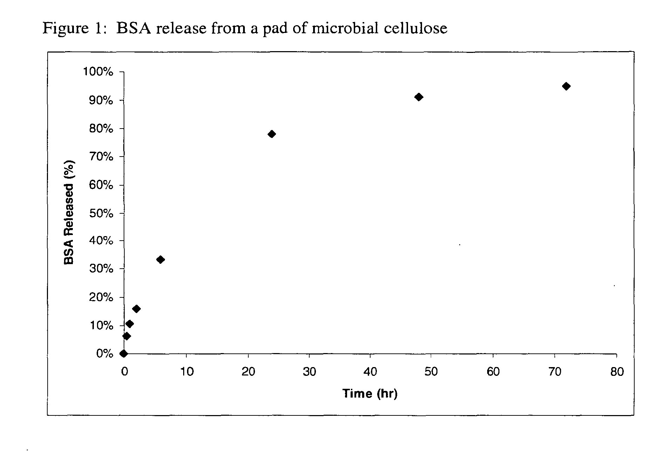

Material can be prepared the same as in Example 1, with the added step of being soaked in a 1% solution of bovine serum albumin (BSA) for 24 hours. Following saturation in the BSA solution the sample is placed in a 0.9% saline solution of 20x the sample mass. Aliquots are removed from the solution at various time points to determine the BSA release profile. The BSA concentration is assessed via ultraviolet/visible spectrophotometry. Figure 1 shows BSA was completely recovered following 72 hours in the saline solution indicating little to no binding of BSA to the microbial cellulose.

### Example 3:

Material can be prepared as per Example 1, however prior to packaging the material can be placed in a Waring Blender with additional water to form a paste. Once processed extra fluid is removed from the material by straining or centrifugation and then packaged. The fiber size can be controlled by blending time to result in materials with different physical properties. Samples containing 5% microbial cellulose were made with processing times of one and five minutes. Stiffness testing was performed with a UNITED Tensile Tester using the circular bend procedure (ASTM test method D 4032). Samples were formed into discs with a diameter of between 3 and 4 cm with a thickness of between 5 and 7 mm. Stiffness values for one and five minutes processing were 5.6±1.7 and 2.3±0.7N, respectively, suggesting larger fiber sizes result in a more cohesive material.

### Example 4:

Material can be prepared as per Example 3, however, an additional flow enhancer may be added to result in a moldable paste. Tween, PEG, and carboxymethyl cellulose have been added to microbial cellulose paste samples in concentrations ranging from 2.5 to 10% to alter the conformability characteristics of the pastes. Furthermore the addition of a flow enhancer results in reduced fluid and/or active ingredient loss during handling.

### Example 5:

A moldable paste containing 2.5% PEG 400, 10% 100-200 nm hydroxyapatite, and approximately 4% microbial cellulose. 500 milligrams of PEG 400 was mixed into 8500 milligrams of a 5% microbial cellulose paste prepared per Example 3. After the PEG and microbial cellulose paste components were well mixed, 1000 milligrams of 100-200 nm hydroxyapatite was mixed into the paste. The resulting paste contained a PEG 400 concentration of 2.5% (w/w), a hydroxyapatite concentration of 10% (w/w), and microbial cellulose concentration of 4% (w/w). This provided a moldable paste with excellent formability properties and moderate water retention properties. A similar paste containing 5% PEG 400, 10% 100-200 nm hydroxyapatite, and 4% microbial cellulose was also prepared. No differences were observed in formability or water retention properties.

### Example 6:

A moldable paste containing 5% carboxymethyl cellulose and 5% microbial cellulose.

519 milligrams of carboxymethyl cellulose (molecular weight = 250,000) was mixed into 9,574 milligrams of a 5% microbial cellulose paste prepared per Example 3. The components were well mixed and allowed to sit for 72 hours at room temperature. This provided a moldable paste with moderate formability properties and excellent water retention properties.

### Example 7:

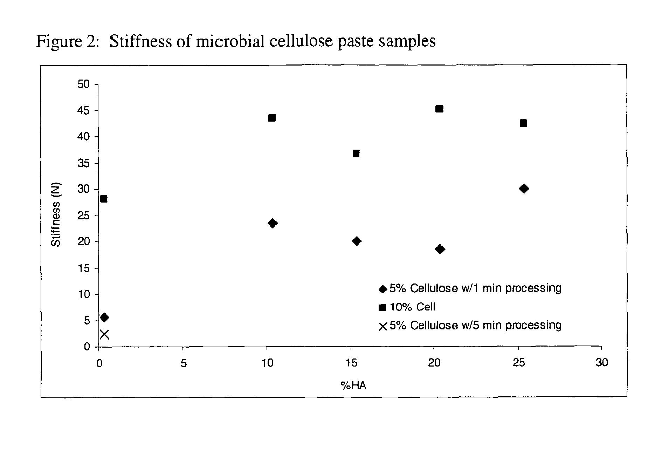

Material can prepared as in Example 4, however additional calcium salts (e.g. hydroxyapatite, tricalcium phosphate, calcium sulfate) can be added to form a composite paste. Paste samples with 5 and 10% microbial cellulose were made by incorporating 10, 15, 20 and 25% hydroxyapatite (HA). Stiffness values (Figure 2) and conformability were used to assess the handling properties of the composites. Stiffness values for composite samples with 5% microbial cellulose increased 3-fold with the addition of 10% HA. Stiffness values remained constant with further increases in HA concentration, although conformability properties decreased. 10% microbial cellulose samples showed a 50% increase in stiffness with 10% HA and as seen with the 5% microbial cellulose samples the stiffness remained constant with increases in HA and similar decreases in conformability were observed.

### Example 8:

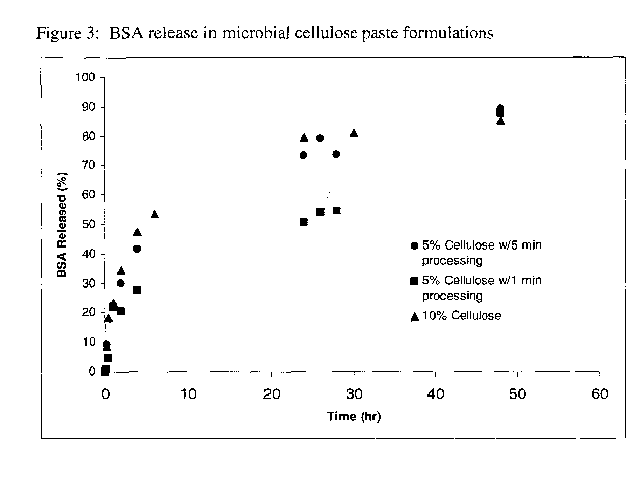

BSA release profiles were determined with materials described in Example 4. Samples were loaded by soaking in a 1% BSA solution for 24 hours. After loading the individual samples were packed into a porous nylon sample bag and then placed in an aqueous solution with a volume 20x the mass of the paste sample. Aliquots were removed at various time points and analyzed with UV/Vis to determine the solution concentration which then could be used to determine the amount of BSA remaining in the paste. Measurements were taken until the solution reached the theoretical equilibrium concentration. Figure 3 shows that the 10% microbial cellulose sample releases BSA at a higher rate than the 5% microbial cellulose sample reaching equilibrium at 30 hours as opposed to 48 hours for the 5% sample. Furthermore the 5% microbial cellulose sample processed for 5 minutes shows a similar profile as the 10% microbial cellulose sample.

### Example 9:

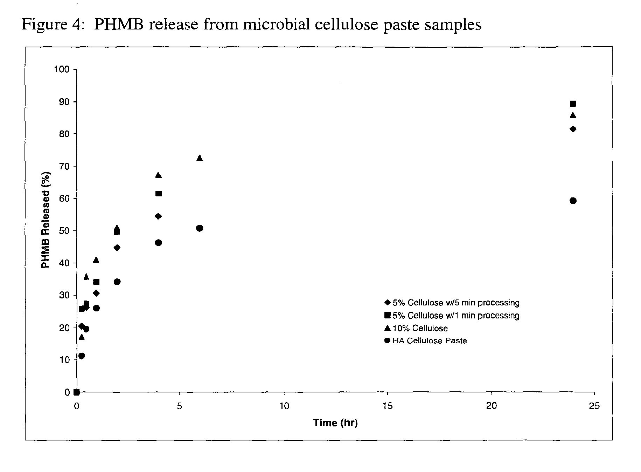

PHMB release profiles were determined with materials described in Example 4 and in addition a 5% microbial cellulose sample with 10% HA was also evaluated. Samples were loaded by soaking in a 1% PHMB solution for 24 hours. After loading the individual samples were packed into a porous nylon sample bag and then placed in an aqueous solution with a volume 20x the mass of the paste sample. Aliquots were removed at various time points and analyzed with UV/Vis to determine the solution concentration which then could be used to determine the amount of PHMB remaining in the paste. Measurements were taken until the solution reached the theoretical equilibrium concentration. Figure 4 shows that the pure microbial cellulose sample released PHMB at a similar rate reaching equilibrium at 24 hours However, the HA containing sample still contains approximately 50% more PHMB at 24 hours indicating binding between HA and PHMB. As seen with the BSA release profile there appears to be little interaction between the microbial cellulose fibers and additive allowing for complete release of additive in a pure microbial cellulose formulation. The addition of HA to the microbial cellulose paste provides a means to control the release profile of the paste if there are specific interactions between additives.

### Example 10:

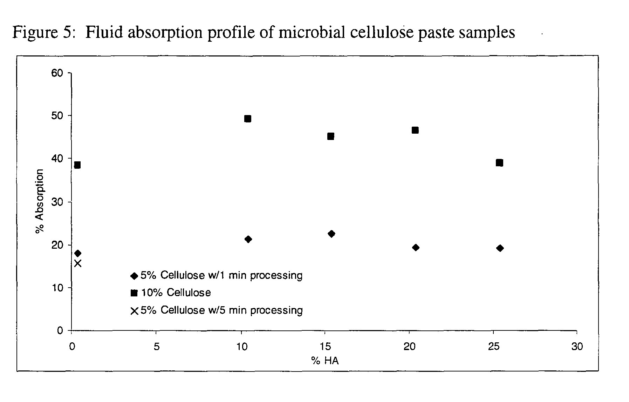

Samples were prepared as described in Example 5 and absorption and donation characteristics of the materials were determined. These samples were tested for absorption from a saturated sponge and donation to a dry surface. For the absorption test, approximately 2.5g of the paste sample was placed on top of a sponge sitting in a 0.9% saline bath at room temperature. The liquid level was maintained at the level of the sponge. Samples were removed after 24hr and reweighed to determine the quantity of saline absorbed by the paste, and the absorption was reported as a percentage of the initial weight of the sample. Figure 5 shows the absorption profile for this set of pastes. As expected the absorption scales with the amount of microbial cellulose present in the paste approximately doubling from 5 to 10% microbial cellulose. Addition of HA results in little if any significant change in the absorption properties of the pastes at both 5 and 10% microbial cellulose contents.

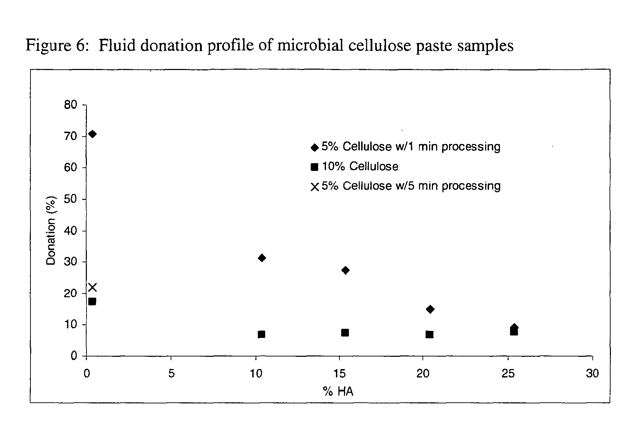

Donation testing was performed by spreading between 2.5 and 3.5g of paste over a circular area on a 3in x 3in piece of pre-weighed smooth leather. Samples were removed after 2hr and the leather was reweighed to determine the quantity of moisture donated to the dry surface. Donation results were reported as a percentage of the initial weight of the sample, and are shown graphically in Figure 6. Donation decreased significantly upon increasing microbial cellulose content to 10% from 5% microbial cellulose. The addition of HA also affected the donation characteristics of the paste most pronounced in the 5% microbial cellulose samples. Donation decreased approximately linearly with the additions of HA to the 5% microbial cellulose samples resulting in an overall 80% decrease with the addition of 25% HA. Samples with 10% microbial cellulose showed a 40% decrease irregardless of HA concentration.

### Example 11:

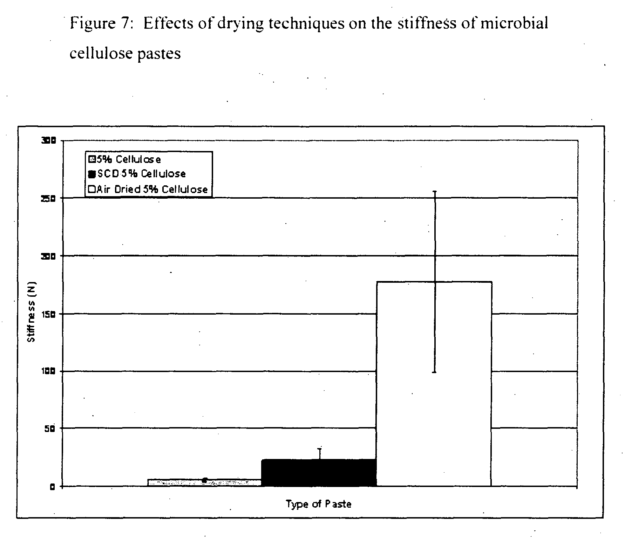

Material can be prepared as described in Example 3 and dried under ambient conditions at elevated temperatures. After drying the resulting material is hard, brittle, and non-conforming. The stiffness of the air-dried material was determined and a 3,200% increase was observed compared to the stiffness of its non-dried counterpart, as shown in Figure 7.

### Example 12:

Material can be prepared as described in Example 3 and dried using a supercritical drying (SCD) process with CO₂. The material first undergoes a solvent exchange process with methanol followed by the SCD processing. Following the SCD process the material is soft and spongy with a stiffness of 22.5±9.6 N in contrast to the hard and brittle material described in Example 11, as shown in Figure 7.

### Example 13:

Microbial cellulose pastes comprised of 5% and 10% microbial cellulose were made per Example 3. Samples containing PEG 400, Tween 80, and hydroxyapatite were prepared using the 5% microbial cellulose paste. The pH of each sample was measured and is shown in Table 1.

**TABLE 1**

| **Microbial cellulose (%)** | **PEG (%)** | **Tween (%)** | **HA (%)** | **pH** |
|---|---|---|---|---|
| 5 | 0 | 0 | 0 | 7.31 |
| 10 | 0 | 0 | 0 | 7.25 |
| 4 | 0 | 0 | 10 | 8.11 |
| 4 | 2.5 | 0 | 10 | 8.20 |
| 4 | 5 | 0 | 10 | 8.27 |
| 4 | 0 | 10 | 0 | 5.30 |

### Example 14:

A 5% microbial cellulose paste was prepared per example 3. Portions of the paste were oxidized by incubating them in 80 mM NaIO₄ with different periodate:microbial cellulose ratios to achieve oxidation levels 10 and 30%. Incubation was conducted in closed reaction vessels within a darkened incubator for 18.5 hrs at 30°C. Following incubation, samples were removed from the oxidizing solutions and placed into extracts of DI water to remove excess NaIO₄. Oxidized pastes with 40%, and 80% β tricalcium phosphate (TCP) by dry weight were prepared. The samples were then supercritically dried per example 12. After drying, sample extracts were prepared by mixing the samples for ≥60 minutes in 26.67 g DI H₂O (pH = 5.33) per gram of the test sample. The pH of each sample extract was measured and is shown in Table 2.

**TABLE 2**

| **Microbial cellulose (%)** | β **TCP (%)** | **Oxidation (%)** | **pH** |
|---|---|---|---|
| 100 | 0 | 10 | 5.80 |
| 60 | 40 | 10 | 6.59 |
| 20 | 80 | 10 | 6.85 |
| 100 | 0 | 30 | 5.97 |
| 60 | 40 | 30 | 6.55 |

### Example 15:

A 5% microbial cellulose paste was prepared per example 3. A sample containing 62% microbial cellulose and 38% hydroxypropyl cellulose (mol wt. = 80,000) by dry weight was prepared using the 5% microbial cellulose paste.

### Example 16:

A 5% microbial cellulose paste was prepared per example 3. A sample containing 37% microbial cellulose, 23% hydroxypropyl cellulose (mol wt. = 80,000), and 40% β tricalcium phosphate (TCP) by dry weight was prepared using the 5% microbial cellulose paste.

### Example 17:

A 5% microbial cellulose paste was prepared per example 3. A sample containing 12% microbial cellulose, 8% hydroxypropyl cellulose (mol wt. = 80,000), and 80% P tricalcium phosphate (TCP) by dry weight was prepared using the 5% microbial cellulose paste.

### Example 18:

Sixteen skeletally mature ewes will be used to evaluate the performance of moldable pastes derived from both resorbable and non-resorbable microbial cellulose. Each ewe will have eight defects created in the tibia and metatarsal bones (four per bone) in the right hind leg. In each animal, two holes will be drilled in the medial side of the proximal and distal tibia and two holes will be drilled in the medial side of the proximal and distal metatarsal bone. Each hole will be 5 mm in diameter and will be created in a manner which minimizes damage to the exposed bone edges. All holes will be -5 mm in depth with no residual underlying cancellous bone left at the implant sites. Stainless marker screws will mark the location of the midpoint of each pair of holes, to aid in subsequent location of each defect at the time of analysis.

With histological analysis, each implant site will be observed for new bone formation, residual implant material and any evidence of pathologic response for each of the materials. Observations will be made at 4 and 8 weeks. Negative controls for the study will be left open; positive controls will use autograft material harvested from the iliac crest at the time of surgery. Moldable paste amples (Table 3 below) will be implanted in the locations given in Table 4 below so as to minimize any potential positional effects and to assure that each test sample has a minimum of six sites per time point. There will be a total of five positive control sites and five negative control sites.

**TABLE 3: Test Articles**

| Sample # | Identification | # of holes |
|---|---|---|
| 1 | Autograft | 6 |
| 2 | BC | 6 |
| 3 | BC + HPC | 6 |
| 4 | BC + HPC + TCP (40%) | 6 |
| 5 | BC + HPC + TCP (80%) | 6 |
| 6 | No implant (negative control) | 6 |
| 7 | RC (10%) + Autograft | 6 |
| 8 | RC (10%) | 6 |
| 9 | RC (30%) | 6 |
| 10 | RC (10%) + TCP (80%) | 5 |
| 11 | RC (30%) + TCP (80%) | 5 |
| Total | | 64 |

| | | |
|---|---|---|
| BC: biosynthesized cellulose which is not resorbable RC: resorbable cellulose. | | |

**TABLE 4: Test Article Locations**

| Implant site | | | | | | | | |
|---|---|---|---|---|---|---|---|---|
| Sheep # | T-VP | T-P | T-D | T-VD | M-VP | M-P | M-D | M-VD |
| 1,9 | 1 | 2 | 3 | 4 | 5 | 6 | 7 | 8 |
| 2, 10 | 9 | 10 | 11 | 1 | 2 | 3 | 4 | 5 |
| 3,11 | 6 | 7 | 8 | 9 | 10 | 11 | 1 | 2 |
| 4,12 | 3 | 4 | 5 | 6 | 7 | 8 | 9 | 10 |
| 5,13 | 11 | 1 | 2 | 3 | 4 | 5 | 6 | 7 |
| 6, 14 | 8 | 9 | 10 | 11 | 1 | 2 | 3 | 4 |
| 7, 15 | 5 | 6 | 7 | 8 | 9 | 10 | 11 | 3 |
| 8, 16 | 21 | 11 | 4 | 5 | 8 | 7 | 10 | 9 |

Where sites are identified as follows:
- T-VP: Tibia, very proximal
- T-P: Tibia, proximal
- T-D: Tibia, distal
- T-VD: Tibia, very distal
- M-VP: Metatarsal, very proximal
- M-P: Metatarsal, proximal
- M-D: Metatarsal, distal
- M-VD: Metatarsal, very distal
where "very distal" and "very proximal" identify those sites toward the extremities of the bones and "distal" and "proximal" describe those sites toward the center of the bone.

## Claims

1. Moldable paste comprising microbial cellulose in an amount of from 1 to 99 % by weight.

2. The paste of claim 1, wherein the microbial cellulose is further chemically modified by oxidation to make it bioresorbable.

3. The paste of one or more of the preceding claims, wherein the paste has a stiffness less than 150 N.

4. The paste of one or more of the preceding claims, wherein the paste comprises an ingredient for flow modification.

5. The paste of claim 4, wherein said ingredient for flow modification is present in an amount of 0.1 to 40 % by weight.

6. The paste of claim 4 or claim 5, wherein the ingredient for flow modification is hydroxypropyl cellulose.

7. The paste of one or more of the preceding claims, further comprising a calcium salt in an amount of 5 to 90 % by weight.

8. The paste of claim 7, wherein the calcium salt is one or more of hydroxyapatite, tricalcium phosphate (α or β phases), and calcium sulfate.

9. The paste of one or more of the preceding claims, which is obtainable by preparing a microbial cellulose pellicle; forming a paste by chopping, grinding or milling the pellicle, subjecting the paste to a solvent exchange process with methanol and subsequently a supercritical drying process with carbon dioxide.

10. The paste of one or more of the preceding claims, further comprising antimicrobial agents, drugs, growth factors or other bioactive agent.
10a. The paste of one or more of the preceding claims that exhibits minimal dispersion upon exposure to a fluid.

11. Use of a paste of one or more of the preceding claims for the manufacture of an agent for promoting hard tissue growth.

12. Use of claim 11, wherein the agent is for bone void filling or a bone graft substitute.
